# FASCICULE DE BREVET EUROPEEN

(11) **EP 3 307 331 B1**
(45) Date de publication et mention de la délivrance du brevet: **17.02.2021**
(21) Numéro de dépôt: 16732986.1
(22) Date de dépôt: 14.06.2016
(51) Int. Cl.: A61L 2/08, B67B 3/00, B65G 47/84

(54) **INSTALLATION DE STERILISATION D'ARTICLES PAR RAYONNEMENT ASSURANT LE DEPLACEMENT ET DES VARIATIONS D'ORIENTATION DES ARTICLES**
EINRICHTUNG ZUM STERILISIEREN VON GEGENSTÄNDEN DURCH STRAHLUNG MIT BEREITSTELLUNG DER BEWEGUNG UND ÄNDERUNGEN DER AUSRICHTUNG DER GEGENSTÄNDE
FACILITY FOR STERILISING ITEMS BY RADIATION PROVIDING THE MOVEMENT AND CHANGES IN THE ORIENTATION OF THE ITEMS

(30) Priorité: 15.06.2015 FR 1555447
(43) Date de publication de la demande: 18.04.2018
(73) Titulaire: Serac Group, 72400 La Ferté Bernard (FR)
(72) Inventeur: GESLOT, Nicolas, 72320 Lamnay (FR)
(74) Mandataire: Lavialle, Bruno François Stéphane
(86) Numéro de dépôt international: PCT/EP2016/063637
(87) Numéro de publication internationale: WO 2016/202802

(56) Documents cités:
- EP-A1- 1 749 747
- EP-A1- 2 149 500
- WO-A1-2009/009681
- JP-A- H11 193 009
- US-A1- 2011 142 731
- US-A1- 2012 134 878
- US-A1- 2013 193 344
- US-A1- 2013 272 920

## Description

La présente invention concerne une installation de stérilisation d'articles, notamment des bouchons ou des bouteilles, par un rayonnement.

### ARRIÈRE-PLAN DE L'INVENTION

On sait que lors de la stérilisation d'articles par rayonnement, notamment par bombardement électronique, il est souhaitable que le rayonnement atteigne directement les surfaces à traiter en raison de l'atténuation que provoque tout obstacle sur la trajectoire du rayonnement.

À cet effet, on connaît du document FR-A-2865135, une installation de stérilisation d'articles comportant deux organes de bombardement électronique disposés selon des orientations différentes par rapport aux articles à stériliser. Ainsi, par un positionnement approprié des organes de bombardement électronique en fonction de la forme des articles à stériliser, il est possible de s'assurer que chaque organe de bombardement électronique est en regard d'une partie de l'article de faible épaisseur, de sorte qu'il est possible d'assurer une stérilisation de chaque partie de l'article avec un bombardement électronique de faible énergie.

Cette installation présente l'inconvénient de nécessiter au moins deux dispositifs générateurs de rayonnement qui sont des équipements lourds tant par leur coût que par les dispositions qui doivent être prises pour éviter des fuites de rayonnement préjudiciable à la santé des opérateurs de l'installation. US2013193344 divulgue à la fois le déplacement des bouchons à stériliser selon une première trajectoire circulaire et leur rotation sur eux-mêmes.

### OBJET DE L'INVENTION

Un but de l'invention est de proposer une installation de stérilisation d'article minimisant le nombre de dispositifs générateurs de rayonnement nécessaires pour assurer une stérilisation satisfaisante des articles.

### BRÈVE DESCRIPTION DE L'INVENTION

En vue de la réalisation de ce but, on propose selon l'invention une installation de stérilisation selon la revendication 1.

Ainsi, des parties des articles qui n'étaient pas soumises au rayonnement pour une première orientation, sont soumises au rayonnement lors des variations de l'orientation des articles, de sorte qu'avec un seul générateur il est possible d'augmenter la surface traitée.

Le dispositif support d'articles comporte un organe de déplacement d'articles, configuré pour assurer un déplacement des articles selon une trajectoire circulaire autour d'un premier axe de rotation et des moyens pour assurer une rotation des articles autour d'un second axe de rotation s'étendant radialement par rapport à la trajectoire circulaire.

Selon un premier mode de réalisation, en relation avec des articles cylindriques de section circulaire, l'installation comporte un bâti fixe comprenant une piste annulaire, et une plateforme rotative qui est montée pour tourner autour d'une direction axiale de la piste annulaire, et qui comporte des alvéoles surplombant la piste annulaire, les alvéoles étant dimensionnées pour qu'un article engagé dans une alvéole repose sur la piste annulaire.

Selon un second mode de réalisation en relation avec des articles cylindriques de section circulaire l'installation comporte un bâti fixe comprenant une piste annulaire, et une plateforme rotative qui est montée pour tourner autour d'une direction axiale de la piste annulaire, et qui comporte une partie annulaire qui surplombe la piste annulaire et qui est en contact avec les articles cylindriques pour les faire tourner autour d'une direction radiale de la piste annulaire.

De préférence, l'installation comporte une première plateforme rotative, et une seconde plateforme rotative, et des moyens de transfert pour transférer les articles de la première plateforme à la seconde plateforme, les moyens de transfert étant agencés pour effectuer une inversion d'une face des articles soumise à un rayonnement.

### BRÈVE DESCRIPTION DES DESSINS

D'autres caractéristiques et avantages de l'invention apparaîtront encore à la lecture de la description qui suit d'un mode de réalisation préféré non limitatif de l'invention en référence aux figures ci-jointes parmi lesquelles:
- la figure 1 est une vue schématique en perspective de l'invention,
- la figure 2 est une vue agrandie et plus détaillée de l'encart II de la figure 1,
- la figure 3 est une vue agrandie et plus détaillée de l'encart III de la figure 1,
- la figure 4 est une vue agrandie et plus détaillée de l'encart IV de la figure 1,
- la figure 5 est une vue agrandie et plus détaillée de l'encart V de la figure 1,
- la figure 6 est une vue schématique, partielle et en perspective d'un mode de réalisation ne faisant pas partie de l'invention, adapté à la stérilisation de bouteilles.

### DESCRIPTION DÉTAILLÉE DE L'INVENTION

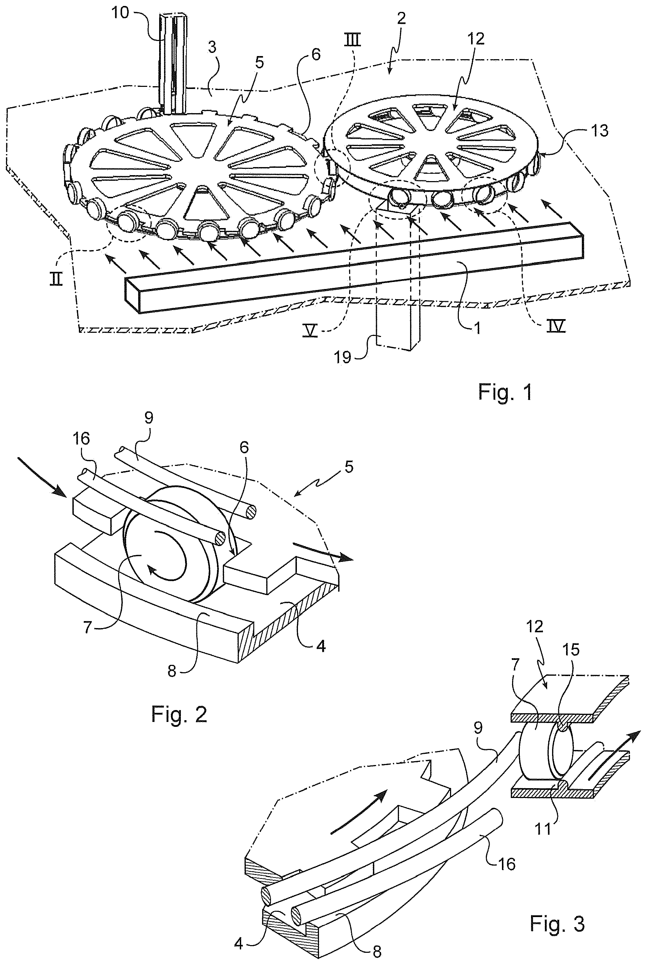

En référence aux figures 1 à 5, l'installation représentée est destinée à assurer une stérilisation de bouchons. À cet effet l'installation comporte un dispositif générateur de rayonnement 1, et un dispositif support d'articles 2 disposé en regard du dispositif générateur de rayonnement.

Le dispositif support d'articles 2 comporte un bâti fixe 3 comprenant une première piste annulaire 4 et une première plate-forme rotative 5 qui est montée pour tourner autour d'une direction axiale de la piste annulaire 4. La plate-forme rotative 5 comporte des alvéoles 6 surplombant la piste annulaire 4. Les alvéoles 6 sont dimensionnées pour qu'un bouchon engagé dans une alvéole repose par la tranche sur la piste annulaire 4. En outre, la plateforme 5 est espacée de la piste 4 de façon que le bord arrière d'une alvéole, par référence au sens de rotation de la plateforme, prenne appui sur un bouchon sensiblement à mi-hauteur de celui-ci, c'est-à-dire au niveau du diamètre horizontal. Sur le côté extérieur de la piste 4 par référence à l'axe de rotation, les alvéoles 6 sont ouvertes et les bouchons 7 sont maintenus dans les alvéoles par un trottoir 8 en saillie le long du bord externe de la piste annulaire. En partie haute, les bouchons sont maintenus par un rail de guidage interne 9 et un rail de guidage externe 16. Ainsi, lors de la rotation de la plateforme 5, les bouchons 7 sont entraînés en rotation autour d'une direction radiale de la piste annulaire 4.

L'accès aux alvéoles par le haut est totalement libre de sorte que pour alimenter la plateforme 5 en bouchons il suffit de prévoir une colonne 10 dans laquelle les bouchons sont empilés sur la tranche, et de monter la colonne 10 sur le bâti de façon qu'elle surplombe les alvéoles 6. Dans le mode de réalisation illustré, les bouchons 7 sont engagés dans les alvéoles 6 avec leur chapeau tourné vers l'extérieur.

L'installation comporte en outre une seconde piste annulaire 11 à laquelle est associée une seconde plate-forme rotative 12.

Comme précédemment, la plate-forme rotative 12 est montée pour tourner autour d'une direction axiale de la piste annulaire 11. Dans ce mode de réalisation, la plate-forme 12 comporte une partie annulaire 13 qui surplombe la piste annulaire 11 et qui est en contact avec les bouchons 7 pour les faire tourner autour d'une direction radiale de la piste annulaire 11. Du côté interne, par référence à une direction radiale de la piste annulaire 11, les bouchons sont maintenus par un trottoir 14 en saillie par rapport à la piste annulaire 11, et par un trottoir 15 en saillie vers le bas par rapport à la partie annulaire 13 de la plate-forme 12. Du côté externe, les bouchons sont maintenus par une glissière 9 supportée par le bâti au moyen de potences non représentées.

Les deux plates-formes 5 et 12 tournent en sens inverse pour permettre un transfert par les rails de guidage 9 et 16. Dans la zone de transfert, le trottoir 8 est interrompu. Dans le mode de réalisation illustré, le rail de guidage externe 16 est également interrompu après la zone de transfert, mais il pourrait être configuré pour s'étendre à l'aplomb de la piste 11 et assurer un guidage interne des bouchons. On remarquera qu'en raison des sens de rotation inverses des plateformes et du transfert direct de la première plateforme à la seconde plateforme, la face des bouchons soumise au rayonnement est inversée lors du transfert. Ceci permet de traiter les deux faces du bouchon avec un seul dispositif générateur de rayonnement. En fin de parcours des bouchons 7, ceux-ci tombent par une ouverture 18 dans la piste annulaire 11 et sont récupérés par une goulotte de transfert 19 qui les achemine vers la zone d'utilisation.

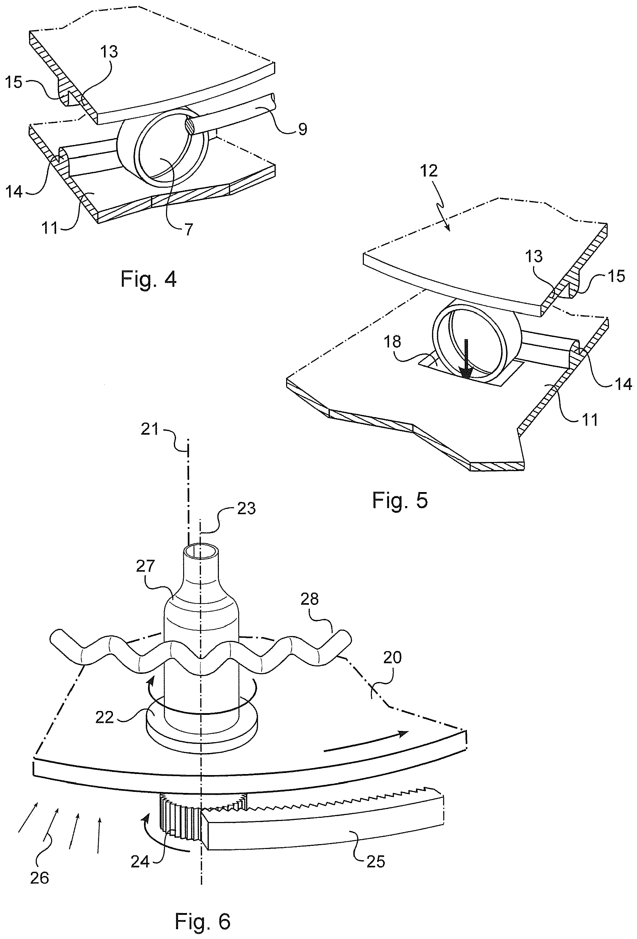

En référence à la figure 6, l'installation comporte une plateforme rotative 20 montée pour tourner autour d'un axe de rotation 21 sur un bâti non représenté. Des platines 22, dont une seule a été illustrée sur la figure, sont portées par la plateforme, sur laquelle elles sont montées pour tourner autour d'un axe de rotation 23 parallèle à l'axe de rotation 21. Chaque platine 22 est associée à un pignon 24 qui engrène avec un secteur denté 25 porté par le bâti dans la zone d'irradiation symbolisée par des flèches 26. Une bouteille 27 est posée sur chaque platine, avec son axe de symétrie en coïncidence avec l'axe de rotation 23. Un guide 28 relié au châssis, retient latéralement la bouteille. Pour éviter que le guide 28 fasse obstacle au rayonnement, le guide 28 a une structure en zigzag dans un plan vertical.

Bien entendu l'invention n'est pas limitée au mode de réalisation décrit et on peut y apporter des variantes de réalisation sans sortir du cadre de l'invention telle que définie par les revendications.

En particulier, bien que l'invention ait été décrite pour la stérilisation de bouchons ou de bouteilles, elle peut être utilisée pour d'autres articles cylindriques section circulaire.

Bien que dans le mode de réalisation illustré les articles soient portés par des plateformes rotatives tournant en sens inverses, on peut réaliser l'invention avec des plateformes rotatives tournant dans le même sens, les moyens de transfert étant adaptés en conséquence pour inverser la face des articles soumise au rayonnement.

Bien que l'invention ait été décrite avec un seul dispositif de bombardement électronique, il est possible que pour certains articles il soit nécessaire de prévoir plusieurs dispositifs de bombardement électronique. Cela ne retire rien à l'intérêt de l'invention qui permet d'augmenter la surface traitée par chacun des dispositifs de bombardement électronique.

## Revendications

1. Installation de stérilisation d'articles cylindriques de section circulaire (7) par rayonnement comprenant :
un dispositif générateur de rayonnement (1) et
un dispositif support d'articles (2) disposé en regard du dispositif générateur de rayonnement,
le dispositif support d'articles (2) comportant un bâti fixe (3) comprenant une piste annulaire (4),
le dispositif support d'articles (2) comportant également une plateforme rotative (5) qui est montée pour tourner autour de l'axe de symétrie de la piste annulaire (4),
la plateforme rotative (5) comportant des alvéoles (6) surplombant la piste annulaire (4) et dimensionnées pour qu'un article cylindrique de section circulaire (7) soit engagé dans une alvéole (6) et repose sur la piste annulaire (4), et que l'axe de symétrie de l'article cylindrique (7) s'étende radialement par rapport à la piste annulaire (4),
la plateforme rotative (5) étant configurée pour assurer le déplacement des articles cylindriques de section circulaire (7) engagés dans les alvéoles (6) avec leur axe de symétrie s'étendant radialement par rapport à la piste annulaire (4) et reposant sur la piste annulaire (4), le déplacement s'effectuant devant le dispositif générateur de rayonnement (1) en tournant la plateforme rotative autour d'un axe de symétrie de la piste annulaire (4).

2. Installation selon la revendication 1, la plateforme rotative (5) étant une première plateforme, et comportant une seconde plateforme rotative (12), et des moyens de transfert (9, 16) pour transférer les articles de la première plateforme (5) à la seconde plateforme (12), les moyens de transfert étant agencés pour effectuer une inversion d'une face des articles soumise à un rayonnement.

3. Installation selon la revendication 2, dans laquelle les deux plates-formes (5, 12) tournent en sens inverses.

4. Installation selon la revendication 2 ou 3, dans laquelle une portion de la plateforme rotative du deuxième dispositif de support d'articles s'étend au-dessus d'une portion de la plateforme rotative du premier dispositif de support d'articles dans une zone de transfert pour transférer directement les articles d'une plateforme à l'autre.

5. Installation selon l'une quelconque des revendications précédentes, dans laquelle la plateforme rotative comporte une partie annulaire (13) qui surplombe la piste annulaire (11) et qui est en contact avec la surface cylindrique des articles.

6. Installation selon l'une quelconque des revendications précédentes, dans laquelle la plateforme (5) est espacée de la piste (4) de façon que le bord arrière d'une alvéole, par référence au sens de rotation de la plateforme, prenne appui sur un article sensiblement à mi-hauteur de celui-ci.

7. Installation selon l'une quelconque des revendications précédentes, dans laquelle le dispositif est agencé de telle manière que l'accès aux alvéoles par le haut soit totalement libre.

## Patentansprüche

1. Anlage zum Sterilisieren von zylindrischen Artikeln (7) mit kreisförmigem Querschnitt durch Strahlung, umfassend:
eine Strahlungserzeugungsvorrichtung (1) und
eine Artikelhaltevorrichtung (2), die gegenüber der Strahlungserzeugungsvorrichtung angeordnet ist,
wobei die Artikelhaltevorrichtung (2) ein ortsfestes Gestell (3) umfasst, das eine ringförmige Bahn (4) enthält,
wobei die Artikelhaltevorrichtung (2) ferner eine Drehplattform (5) umfasst, die um die Symmetrieachse der ringförmigen Bahn (4) drehbar gelagert ist,
wobei die Drehplattform (5) Zellen (6) umfasst, die sich über der ringförmigen Bahn (4) befinden und so bemessen sind, dass ein zylindrischer Artikel (7) mit kreisförmigem Querschnitt in eine Zelle (6) eingefügt ist und auf der ringförmigen Bahn (4) aufliegt, und dass sich die Symmetrieachse des zylindrischen Artikels (7) radial in Bezug auf die ringförmige Bahn (4) erstreckt,
wobei die Drehplattform (5) ausgebildet ist, um das Bewegen der zylindrischen Artikel (7) mit kreisförmigem Querschnitt, die in die Zellen (6) eingefügt sind, während sich ihre Symmetrieachse radial in Bezug auf die ringförmige Bahn (4) erstreckt und sie auf der ringförmigen Bahn (4) aufliegen, sicherzustellen, wobei die Bewegung vor der Strahlungserzeugungsvorrichtung (1) erfolgt, indem die Drehplattform um eine Symmetriesachse der ringförmigen Bahn (4) gedreht wird.

2. Anlage nach Anspruch 1, wobei die Drehplattform (5) eine erste Plattform ist, und umfassend eine zweite Drehplattform (12) und Übertragungsmittel (9, 16) zum Übertragen der Artikel von der ersten Plattform (5) auf die zweite Plattform (12), wobei die Übertragungsmittel so ausgebildet sind, dass sie eine Umkehrung einer Seite der Artikel, die einer Strahlung ausgesetzt ist, bewirken.

3. Anlage nach Anspruch 2, bei der sich die beiden Plattformen (5, 12) in umgekehrte Richtungen drehen.

4. Anlage nach Anspruch 2 oder 3, bei der sich ein Abschnitt der Drehplattform der zweiten Artikelhaltevorrichtung über einem Abschnitt der Drehplattform der ersten Artikelhaltevorrichtung in einer Übertragungszone zum direkten Übertragen der Artikel von einer Plattform zur anderen erstreckt.

5. Anlage nach einem der vorhergehenden Ansprüche, bei der die Drehplattform einen ringförmigen Teil (13) umfasst, der sich über der ringförmigen Bahn (11) befindet und der in Kontakt mit der zylindrischen Fläche der Artikel ist.

6. Anlage nach einem der vorhergehenden Ansprüche, bei der die Plattform (5) von der Bahn (4) derart beabstandet ist, dass der hintere Rand einer Zelle, in Bezug auf die Drehrichtung der Plattform, an einem Artikel im Wesentlichen auf halber Höhe desselben zur Anlage kommt.

7. Anlage nach einem der vorhergehenden Ansprüche, bei der die Vorrichtung derart ausgebildet ist, dass der Zugang zu den Zellen von oben vollkommen frei ist.

## Claims

1. Installation for sterilizing by radiation cylindrical articles of circular section (7), the installation comprising:
a radiation-generator device (1) and
an article support device (2) disposed facing the radiation-generator device,
the article support device (2) comprising a stationary frame (3) including an annular track (4),
the article support device (2) also comprising a rotary platform (5) that is mounted to turn about the axis of symmetry of the annular track (4),
the rotary platform (5) comprising recesses (6) overlying the annular track (4) and being dimensioned so that a cylindrical article of circular section (7) is engaged in a recess (6) and rests on the annular track (4), and that the axis of symmetry of the cylindrical article of circular section (7) extends radially with respect to the annular track (4),
the rotary platform (5) being arranged for moving the cylindrical articles of circular section (7) that are engaged in the recesses (6) with their axis of symmetry extending radially with respect to the annular track (4) and that rest on the annular track (4), the move being realized in front of the radiation-generator device (1) by rotating the rotary platform (5) about an axis of symmetry of the annular track (4).

2. Installation according to claim 1, the rotary platform (5) being a first platform, and comprising a second rotary platform (12), and transfer means (9, 16) for transferring the articles from the first platform (5) to the second platform (12), the transfer means being arranged to swap over the faces of each article that are subjected to radiation.

3. Installation of claim 2, wherein the platforms (5, 12) rotate in opposite directions.

4. Installation of claim 2 or 3, wherein a portion of the platform of the second article device extends above a portion of the rotary platform of the first article support device in a transfer zone for directly transferring the articles from one of the platforms to the other.

5. Installation according to anyone of the preceding claims, wherein the rotary platform has an annular portion (13) overlying the annular track (11) and in contact with the cylindrical surface of the articles.

6. Installation according to anyone of the preceding claims, wherein the platform (5) is spaced apart from the track (4) so that recesses have a rear edge, relative to the direction of rotation of the platform, coming to bear against the cap substantially half way up it.

7. Installation according to anyone of the preceding claims, wherein the device is arranged so that an access to the recesses from the top is totally free.
